(19) Europäisches Patentamt
European Patent Office
Office européen des brevets

(11) **EP 3 185 960 B1**

(12) **EUROPÄISCHE PATENTSCHRIFT**

(45) Veröffentlichungstag und Bekanntmachung des Hinweises auf die Patenterteilung:
**17.10.2018 Patentblatt 2018/42**

(21) Anmeldenummer: **15730805.7**

(22) Anmeldetag: **24.06.2015**

(51) Int Cl.:
*A61Q 5/06* (2006.01)    *A61K 8/81* (2006.01)

(86) Internationale Anmeldenummer:
**PCT/EP2015/064174**

(87) Internationale Veröffentlichungsnummer:
**WO 2016/030042 (03.03.2016 Gazette 2016/09)**

(54) **VERWENDUNG EINER KOMBINATION VON AQUASTYLE SH-100 UND ACUDYNE 1000**

USE OF A COMBINATION OF AQUASTYLE SH-100 AND ACUDYNE 1000

UTILISATION D'UNE ASSOCIATION D'AQUASTYLE SH-100 ET D'ACUDYNE 1000

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.08.2014 DE 102014217207**

(43) Veröffentlichungstag der Anmeldung:
**05.07.2017 Patentblatt 2017/27**

(73) Patentinhaber: **Henkel AG & Co. KGaA**
**40589 Düsseldorf (DE)**

(72) Erfinder:
• **LANGE, Julia Bibiane**
**24641 Sievershütten (DE)**
• **RICHTERS, Bernd**
**21129 Hamburg (DE)**
• **BERMUDEZ AGUDELO, Maria Catalina**
**22089 Hamburg (DE)**
• **MARTINEZ, Cyrielle**
**22765 Hamburg (DE)**

(56) Entgegenhaltungen:
WO-A1-2015/095870    WO-A2-2012/054278
DE-A1-102011 081 814    US-A1- 2002 192 179

US-A1- 2012 207 696    US-A1- 2013 344 019

• Anonymous: "Ashland brings performance and style to crystal clear gel with AquaStyle(TM) (NYSE:ASH)", , 1. April 2014 (2014-04-01), XP055205095, Gefunden im Internet: URL:http://investor.ashland.com/releasedetail.cfm?releaseid=836949 [gefunden am 2015-07-28]
• "Innovation Zone 2014", , 1. April 2014 (2014-04-01), Seiten 1-39, XP055205044, Gefunden im Internet: URL:http://www.in-cosmetics.com/RXUK/RXUK_InCosmetics/2015-Website/Reviewpage_downloads/InnovationZoneGuide2014_lowres.pdf?v=635333310957041007 [gefunden am 2015-07-28]
• "ACUDYNE (TM) 1000 Hair Styling Polymer Delivering Outstanding Hold and Durability", , 15. Oktober 2011 (2011-10-15), XP055205325, Gefunden im Internet: URL:http://msdssearch.dow.com/PublishedLiteratureDOWCOM/dh_0880/0901b8038088029b.pdf?filepath=personalcare/pdfs/noreg/324-00438.pdf&fromPage=GetDoc [gefunden am 2015-07-29]

Bemerkungen:
Die Akte enthält technische Angaben, die nach dem Eingang der Anmeldung eingereicht wurden und die nicht in dieser Patentschrift enthalten sind.

EP 3 185 960 B1

**Beschreibung**

Die vorliegende Erfindung betrifft eine kosmetische Zusammensetzung zur Haarfestigung bzw. zur temporären Umformung von keratinischen Fasern, insbesondere menschlichen Haaren, wobei die Zusammensetzung eine Kombination von zwei anionischen Acrylatharzen enthält.

Die temporäre Gestaltung von Frisuren für einen längeren Zeitraum bis hin zu mehreren Tagen erfordert in der Regel die Anwendung festigender Wirkstoffe. Daher spielen Haarbehandlungsmittel, die einer temporären Formgebung der Haare dienen, eine wichtige Rolle. Entsprechende Mittel zur temporären Verformung enthalten als festigenden Wirkstoff üblicherweise synthetische Polymere und/oder Wachse. Mittel zur Unterstützung der temporären Umformung keratinhaltiger Fasern können beispielsweise als Haarspray, Haarwachs, Haargel, Haarschaum konfektioniert werden.

Die wichtigste Eigenschaft eines Mittels zur temporären Verformung von Haaren, im Folgenden auch Stylingmittel genannt, besteht darin, den behandelten Fasern in der neu modellierten Form - d.h. einer den Haaren aufgeprägten Form - einen möglichst starken Halt zu geben. Man spricht auch von starkem Frisurenhalt oder vom hohen Haltegrad des Stylingmittels. Der Frisurenhalt wird im Wesentlichen durch die Art und Menge des eingesetzten festigenden Wirkstoffe bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit (tack) und ein ausgewogener Konditioniereffekt. Weiterhin soll ein Stylingmittel möglichst für alle Haartypen universell einsetzbar und mild zu Haar und Haut sein.

Um den unterschiedlichen Anforderungen gerecht zu werden, wurden als festigende Wirkstoffe bereits eine Vielzahl von synthetischen Polymeren entwickelt, die in Stylingmitteln zur Anwendung kommen. Die Polymere lassen sich in kationische, anionische, nichtionische und amphotere festigende Polymere unterteilen. Idealerweise ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen. Ähnliche Probleme ergeben sich, wenn Wachse als festigender Wirkstoff im Stylingmittel Einsatz finden. Handelt es sich bei dem Stylingmittel um ein Gel oder eine Paste, sollen die Polymere zudem verdickende Eigenschaften besitzen.

Eine bekannte Klasse anionischer Polymere, die in Haarfestigungsprodukten Anwendung finden, sind anionische Acrylatharze. EP 1719499 B1, EP 1719500 B1 und EP 1726331 B1beschreiben derartige Acrylatharze mit der INCI-Bezeichnung Acrylates/Hydroxy Ester Acrylates Copolymer und deren Anwendung in Stylingmitteln, auch in Kombination mit anderen Acrylatcopolymeren. Die in diesen Druckschriften beschriebenen anionischen Acrylatcopolymere sind im Handel z. B. unter der Bezeichnung Acudyne® 180 (The Dow Chemical Company) erhältlich.

WO 2012/054278 A2 erwähnt ebenfalls Acrylates/Hydroxy Ester Acrylates Copolymere als haarfestigende Polymere und verwendet als Beispiel Acudyne® 1000 (The Dow Chemical Company) in Haarschäumen.

Weiterhin sind hydrophob modifizierte Acrylatcopolymere im Handel erhältlich (INCI: Acrylates Copolymer (and) Water), die im Wesentlichen als Verdickungsmittel wirken. Das Datenblatt AquaStyle® SH-100 Polymer (Ashland Inc.) beschreibt ein solches Acrylatcopolymer und dessen Verwendung in Kombination mit Carbomeren. Es wird eine Eignung für kristallklare Haargele, eine gute Anfangssteifheit, Feuchtigkeitsbeständigkeit und eine Langzeitwirkung beschrieben.

Eine Aufgabe der vorliegenden Erfindung war es, weitere geeignete Polymerkombinationen zur Verfügung zu stellen, welche sich durch gute filmbildende und/oder festigende Eigenschaften auszeichnen, einen sehr hohen Haltegrad besitzen ohne dass dabei auf Flexibilität und gute Feuchtebeständigkeit - insbesondere Schweiß- und Wasserbeständigkeit - verzichtet werden müsste und sich zudem für die Herstellung stabil viskoser sowie stabil transparenter kosmetischer Zusammensetzungen eignen. Insbesondere sind derzeit erhältliche Stylingmittel noch dahingehend verbesserbar, dass eine gute Kombination aus Steifheit (Stiffness) und Langzeithalt nicht immer ausreichend gewährleistet wird. Es ist daher eine Aufgabe der vorliegenden Erfindung, derartige Stylingmittel bereitzustellen, die neben den oben genannten Eigenschaften insbesondere sowohl eine gute Steifheit als auch einen guten Langzeithalt ergeben.

Dies wurde erfindungsgemäß durch eine Kombination zweier bestimmter anionischer Acrylatharze erreicht.

Durch die vorliegende Erfindung wird bereitgestellt:

1. Eine kosmetische Zusammensetzung zur temporären Umformung keratinischer Fasern, welche enthält:

Gew. 0.1 - 1.5% mindestens eines anionisches Acrylatcopolymer (a), welches zumindest aus folgenden Monomereinheiten aufgebaut ist: mindestens einer (Meth)Acrylsäureeinheit (a1), mindestens einer (Meth)Acrylsäurealkylestereinheit (a2) und mindestens einer (Meth)Acrylsäurehydroxyalkylestereinheit (a3), und Gew. 0.3 -2.0% mindestens ein anionisches Acrylatcopolymer (b), welches zumindest aus folgenden Monomereinheiten aufgebaut ist: mindestens einer (Meth)Acrylsäureeinheit (b1), mindestens einer (Meth)Acrylsäureethylestereinheit (b2) und mindestens einer (Meth)Acrylsäureestereinheit (b3), die von der (Meth)Acrylsäureethylestereinheit (b2) verschieden ist und eine hydrophobe Gruppe als Estergruppe aufweist, und 0,05 - 1,5 Gew.% Homopolyacrylsäure.

2. Kosmetische Zusammensetzung nach 1, wobei das anionische Acrylatcopolymer (a) ein Molekulargewicht von 130000 bis 160000 aufweist.

3. Kosmetische Zusammensetzung nach 1 oder 2, wobei das anionische Acrylatcopolymer (a) Hydroxyethyl(meth)acrylat als Monomereinheit (a3) aufweist.

4. Kosmetische Zusammensetzung nach einem der Punkte 1 bis 3, wobei das anionische Acrylatcopolymer (a) bei einem Feststoffgehalt von 44 bis 46 Gew.-% und einem pH von 3,30 bis 4,30 bei 25°C eine Viskosität von maximal 150 cPS aufweist (Brookfield LV, Spindel 1,60 UpM).

5. Kosmetische Zusammensetzung nach einem der Punkte 1 bis 4, wobei das anionische Acrylatcopolymer (b) einen (Meth)Acrylsäurealkylester als Monomereinheit (b3) aufweist.

6. Kosmetische Zusammensetzung nach einem der Punkte 1 bis 5, wobei das anionische Acrylatcopolymer (b) Methacrylsäure als Monomereinheit (b1) und Ethylacrylat als Monomereinheit (b2) aufweist.

7. (Bereits in Punkt 1 enthalten).

8. Kosmetische Zusammensetzung einem der vorhergehenden Punkte, wobei das anionische Acrylatcopolymer (b) bei einem Feststoffgehalt von 2 Gew.-% in einer wässerigen neutralisierten Lösung bei 25°C eine Viskosität von 60000 bis 120000 cPs aufweist.

9. Kosmetische Zusammensetzung einem der vorhergehenden Punkte, wobei das anionische Acrylatcopolymer (a) eines mit der INCI-Bezeichnung Acrylates / Hydroxyesters Acrylates Copolymer, insbesondere Acudyne™ 1000 (Dow), ist.

10. Kosmetische Zusammensetzung einem der vorhergehenden Punkte, wobei das anionische Acrylatcopolymer (b) eines mit der INCI-Bezeichnung Acrylates Copolyme (and) Water, insbesondere AquaStyle™ SH-100 (Ashland Inc.), ist.

11. Kosmetische Zusammensetzung einem der vorhergehenden Punkte, wobei das anionische Acrylatcopolymer (a) eines mit der INCI-Bezeichnung Acrylates / Hydroxyesters Acrylates Copolymer und das anionische Acrylatcopolymer (b) eines mit der INCI-Bezeichnung Acrylates Copolymer (and) Water ist.

12. Kosmetische Zusammensetzung einem der vorhergehenden Punkte, wobei das anionische Acrylatcopolymer (a) Acudyne™ 1000 (The Dow Chemical Company) ist und das anionische Acrylatcopolymer (b) AquaStyle™ SH-100 (Ashland Inc.) ist.

13. (Bereits in vorhergehenden Punkten enthalten).

14. Kosmetische Zusammensetzung nach einem der vorhergehenden Punkte, enthaltend, bezogen auf das Gesamtgewicht der kosmetischen Zusammensetzung:

0,2 bis 0,8 Gew.-% des anionischen Acrylatcopolymers (a), und
0,8 bis 1,4 Gew.-% des anionischen Acrylatcopolymers (b).

15. (Bereits in vorhergehenden Punkten enthalten).

16. Kosmetische Zusammensetzung nach einem der vorhergehenden Punkte, wobei die Zusammensetzung als Haargel, Haarspray, Haarschaum oder Haarwachs, insbesondere als Haargel, vorliegt.

17. Verwendung einer kosmetischen Zusammensetzung nach einem der Punkte 1 bis 16 zur temporären Umformung keratinischer Fasern.

18. Verfahren zur temporären Verformung von keratinischen Fasern, insbesondere menschlichen Haaren, bei dem die kosmetische Zusammensetzung nach einem der Punkte 1 bis 16 auf keratinische Fasern appliziert wird.

Es wurde im Rahmen der vorliegenden Erfindung überraschend festgestellt, dass durch Kombination zweier an sich bekannter anionischer Acrylatcopolymere eine verbesserte Kombination aus Langzeitwirkung und Steifheit von Stylingprodukten, insbesondere von Haargelen, erhalten werden kann. Eine derartige Kombination von guter Steifheit und gutem Langzeithalt war nicht erwartbar.

Der Begriff keratinische Fasern umfasst erfindungsgemäß Pelze, Wolle und Federn, insbesondere aber menschliche Haare.

Die wesentlichen Bestandteile der erfindungsgemäßen kosmetischen Zusammensetzung sind das anionisches Acrylatcopolymer (a) und das anionische Acrylatcopolymer (b).

Das anionische Acrylatcopolymer (a) ist zumindest aus folgenden Monomereinheiten aufgebaut: mindestens einer (Meth)Acrylsäureeinheit (a1), mindestens einer (Meth)Acrylsäurealkylestereinheit (a2) und mindestens einer (Meth)Acrylsäurehydroxyalkylestereinheit (a3).

Das Copolymer (a) kann erfindungsgemäß aus weitere Monomereinheiten aufgebaut sein. Gemäß Ausführungsformen der Erfindung ist das Copolymer (a) aber nur aus den Einheiten (a1), (a2) und (a3) aufgebaut, d. h. es besteht aus von diesen Monomereinheiten abgeleiteten Einheiten.

Die mindestens eine Methacrylsäureeinheit (a1) kann eine Methacrylsäure- oder Acrylsäureeinheit sein.

Der Alkylrest der (Meth)Acrylsäurealkylestereinheit (a2) ist bevorzugt ein C1-C8-Alkylrest, der linear oder verzweigt sein kann. Beispiele für Alkylreste sind Methyl, Ethyl, Propyl, Isopropyl, 1-Butyl, 2-Butyl, iso-Butyl,tert-Butyl, lineares oder verzweigtes Pentyl, lineares oder verzweigtes Hexyl, lineares oder verzweigtes Heptyl und lineares oder verzweigtes Octyl. Bevorzugter ist die Alkylgruppe eine C1- bis C5-Alkylgruppe. Gemäß einer Ausführungsform der Erfindung sind zwei oder mehr (Meth)Acrylsäurealkylestereinheiten (a2) enthalten, die sich hinsichtlich der Kohlenstoffzahl der Alkylgruppe unterscheiden. Beispielsweise sind eine C1-C3-Alkylmethacrylateinheit und eine C2-C5-Alkylacrylateinheit enthalten.

Der Hydroxyalkylrest der (Meth)Acrylsäurehydroxyalkylestereinheit (a3) kann ein Hydroxy-C1-C10-Alkylrest sein, bevorzugt ein Hydroxy-C2-C5-Alkylrest. In einer bevorzugten Ausführungsform ist die (Meth)Acrylsäurehydroxyalkylestereinheit (a3) (Meth)Acrylsäurehydroxyethylester.

Der Anteil der Einheiten (a1), (a2) und (a3) in dem Acrylatharz (a) kann in weiten Grenzen variieren. Der Anteil der Einheit (a1) in dem Acrylatcopolymer ist bevorzugt 2 bis 50 Gew.-%, bevorzugter 5 bis 30 Gew.-%. Der Anteil der Einheit (a2) in dem Acrylatcopolymer ist bevorzugt 5 bis 95 Gew.-%, bevorzugter 45 bis 90 Gew.-%. Der Anteil der Einheit (a3) in dem Acrylatcopolymer ist bevorzugt 2 bis 70 Gew.-%, bevorzugter 5 bis 30 Gew.-%.

Das Gewichtsmittel des Molekulargewichts des anionischen Acrylatcopolymers ist bevorzugt 130000 bis 160000, bevorzugter 140000 bis 150000, bestimmt mittels Gelpermeationschromtographie (GPC).

Die Viskosität des in der kosmetischen Zusammensetzung verwendeten anionischen Acrylatcopolymers (a) bei einem Feststoffgehalt von 44 bis 46 Gew.-% und einem pH von 3,30 bis 4,30 bei 25°C ist bevorzugt höchstens 150 cPS (Brookfield LV, Spindel 1, 60 UpM).

Geeignete anionische Acrylatcopolymere (a) sind im Handel unter der INCI-Bezeichnung Acrylates / Hydroxyesters Acrylates Copolymer erhältlich. Am bevorzugtesten ist das anionische Acrylatcopolymer (a) Acudyne® 1000 von The Dow Chemical Company. Dieses hat in der im Handel erhältlichen Form einen Feststoffgehalt von etwa 44 bis 46 Gew.-% und einen pH-Wert von 3.3-4.3.

Das anionische Acrylatcopolymer (b) ist zumindest aus folgenden Monomereinheiten aufgebaut: mindestens einer (Meth)Acrylsäureeinheit (b1), mindestens einer (Meth)Acrylsäureethylestereinheit (b2) und mindestens einer (Meth)Acrylsäureestereinheit (b3), die von der (Meth)Acrylsäureethylestereinheit (b2) verschieden ist und eine hydrophobe Gruppe als Estergruppe aufweist.

Das Copolymer (b) kann erfindungsgemäß aus weiteren Monomereinheiten aufgebaut sein. Gemäß Ausführungsformen der Erfindung ist das Copolymer (a) aber nur aus den Einheiten (b1), (b2) und (b3) aufgebaut, d. h. es besteht aus von diesen Monomereinheiten abgeleiteten Einheiten.

Die mindestens eine (Meth)Acrylsäureeinheit (b1) kann eine Methacrylsäure- oder Acrylsäureeinheit sein, wobei eine Methacrylsäureeinheit bevorzugt ist.

Die mindestens eine (Meth)Acrylsäureethylestereinheit (b2) kann eine Methacrylsäureethylestereinheit oder eine Acrylsäureethylestereinheit, wobei eine Acrylsäureethylestereinheit bevorzugt ist.

Die mindestens eine (Meth)Acrylsäureestereinheit (b3) kann erfindungsgemäß eine (Meth)Acrylsäurealkylestereinheit sein. Die Alkylgruppe der (Meth)Acrylsäurealkylestereinheit dient zur Steuerung der Hydrophobizität des Copolymers. Die Alkylgruppe ist bevorzugt eine lineare oder verzweigte Alkylgruppe mit 2 bis 30 Kohlenstoffatomen, bevorzugter 3 bis 12 Kohlenstoffatomen.

Die hydrophobe Gruppe kann erfindungsgemäß auch eine andere hydrophobe als eine Alkylgruppe sein, z. B. eine aromatische Kohlenwasserstoffestergruppe. Als Beispiel sei eine substituierte oder unsubstituierte Phenylestergruppe oder substituierte oder unsubstituierte Alkylenphenylestergruppe genannt, z. B. eine Benzylestergruppe.

Die Viskosität des in der kosmetischen Zusammensetzung verwendeten anionischen Acrylatcopolymers (b) bei einem Feststoffgehalt von 2 Gew.-% und neutralisierter Lösung bei 25°C ist bevorzugt höchstens 60000 bis 120000 cPS.

Geeignete anionische Acrylatcopolymere (b) sind im Handel unter der INCI-Bezeichnung Acrylates Copolymer (and) Water erhältlich.

Am bevorzugtesten ist das anionische Acrylatcopolymer (b) AquaStyle® SH-100 Polymer von Ashland, Inc. Dieses hat in der im Handel erhältlichen Form einen Feststoffgehalt von etwa 28 bis 32 Gew.-% und einen pH-Wert von 2.1-4.0.

Die kosmetische Zusammensetzung der vorliegenden Erfindung enthält das Acrylatcopolymer (a) und Acrylatcopolymer (b) in für Stylingmittel üblichen und geeigneten Mengen, die für die spezielle Anwendung und Konfektionierung angepasst werden können.

Die erfindungsgemäße kosmetische Zusammensetzung enthält das Acrylatcopolymer (a), bezogen auf das Gesamtgewicht der kosmetischen Zusammensetzung, z. B. in einer Menge von 0,1 bis 5 Gew.-%, bevorzugt 0,1 bis 1,5 Gew.-%, weiterhin bevorzugt 0,2 bis 0,8 Gew.-%, jeweils angegeben als Feststoffgehalt aktiver Substanz in der kosmetischen Zusammensetzung.

Die erfindungsgemäße kosmetische Zusammensetzung enthält das Acrylatcopolymer (b), bezogen auf das Gesamtgewicht der kosmetischen Zusammensetzung, z. B. in einer Menge von 0,1 bis 5 Gew.-%, bevorzugt 0,3 bis 2,0 Gew.-%, weiterhin bevorzugt 0,8 bis 1,4 Gew.-%, jeweils angegeben als Feststoffgehalt aktiver Substanz in der kosmetischen Zusammensetzung.

In einer besonders bevorzugten Ausführungsform der vorliegenden Erfindung enthält die kosmetische Zusammensetzung als das anionische Acrylatcopolymer (a) das im Handel unter der Bezeichnung Acudyne™ 1000 erhältliche Copolymer und als das anionische Acrylatcopolymer (b) das im Handel unter der Bezeichnung AquaStyle™ SH-100 erhältliche Copolymer. Bei dieser Kombination wurden besonders gute Ergebnisse hinsichtlich einer Kombination von Steifheit und Langzeithalt erzielt. Besonders vorteilhaft ist diese Polymerkombination bei Stylingprodukten in Gelform.

Weitere allgemein geforderte Eigenschaften von Stylingprodukten, wie z. B. Feuchtebeständigkeit und niedrige Klebrigkeit, werden insbesondere mit dieser Kombination gleichfalls erzielt, besonders bei Konfektionierung als Haargel.

Bevorzugt enthält die kosmetische Zusammensetzung der vorliegenden Erfindung eine oder mehrere weitere, als Verdickungsmittel oder Gelbildner wirkende Komponente(n), die von dem Acrylatcopolymer (a) und (b) verschieden ist/sind und ebenfalls die Filmbildung unterstützen. Beispiele sind kationische, anionische, nichtionische oder amphotere Polymere. Der Gewichtsanteil dieser weiteren Komponente am Gesamtgewicht der kosmetischen Zusammensetzung kann aufgrund der Anwesenheit der Komponenten (a) und (b) vergleichsweise niedrig sein und beträgt beispielsweise 0,02 bis 3 Gew.-%, bevorzugt 0,05 bis 1,5 Gew.-% und noch bevorzugter 0,2 bis 0,8 Gew.-%.

Beispiele sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer.

Beispiele für nichtionische Polymere sind:

- VinylpyrrolidonVinylester-Copolymere, wie sie beispielsweise unter dem Warenzeichen Luviskol(BASF) vertrieben werden. LuviskolVA 64 und LuviskolVA 73, jeweils Vinylpyrrolidon/Vinylacetat-Copolymere, sind bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminalund Benecel (AQUALON) vertrieben werden.
- Schellack.
- Polyvinylpyrrolidone, wie sie beispielsweise unter der Bezeichnung Luviskol (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone.

Als Gelbildner wirkende Komponente: eine Homopolyacrylsäure (INCI: Carbomer), die im Handel unter dem Namen Carbopol® in unterschiedlichen Ausführungen erhältlich ist. Das Carbomer ist in einem Anteil von 0,05 bis 1,5 Gew.-% und noch bevorzugter 0,2 bis 0,8 Gew.-%, in Bezug auf das Gesamtgewicht der kosmetischen Zusammensetzung, enthalten.

Die erfindungsgemäße kosmetische Zusammensetzung kann weitere übliche Stoffe von Stylingprodukten enthalten. Als weitere geeignete Hilfs- und Zusatzstoffe sind insbesondere zusätzliche Pflegestoffe zu nennen.

Als Pflegestoff kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Wie auch der Zusatz von Glycerin und/oder Propylenglykol erhöht der Zusatz von Panthenol die Flexibilität des bei Anwendung des erfindungsgemäßen Mittels gebildeten Polymerfilms.

Als Pflegestoff können die erfindungsgemäßen Mittel weiterhin mindestens einen Pflanzenextrakt, aber auch Mono- bzw. Oligosaccharide und/oder Lipide enthalten.

Weiterhin sind als Pflegestoff Ölkörper geeignet. Zu den natürlichen und synthetischen kosmetischen Ölkörpern sind beispielsweise zu zählen pflanzliche Öle, flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen. Bevorzugte erfindungsgemäße kosmetische Mittel enthalten mindestens einen Ölkörper, vorzugsweise mindestens einen Ölkörper aus der Gruppe der Silikonöle. Zur Gruppe der Silikonöle zählen insbesondere die Dimethicone, zu welchen auch die Cyclomethicone zu rechnen sind, die aminofunktionellen Silikone sowie die Dimethiconole. Die Dimethicone können sowohl linear als auch verzweigt als auch cyclisch oder cyclisch und verzweigt sein. Geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Esteröle, das heißt Ester von 6-C30-Fettsäuren mit C2-C30-Fettalkoholen, vorzugsweise Monoester der Fettsäuren mit Alkoholen mit 2 bis 24 C-Atomen wie beispielsweise Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V) sind weitere bevorzugte pflegende Ölkörper.

Als Pflegestoffe eignen sich weiterhin Dicarbonsäureester, symmetrische, unsymmetrische oder cyclische Ester der Kohlensäure mit Fettalkoholen, Trifettsäureester von gesättigten und/oder ungesättigten linearen und/oder verzweigten Fettsäuren mit Glycerin oder Fettsäurepartialglyceride, worunter Monoglyceride, Diglyceride und deren technische Gemische zu verstehen sind.

Weiterhin sind in der erfindungsgemäßen Zusammensetzung bevorzugt Emulgatoren bzw. oberflächenaktive Mittel enthalten. Bevorzugt sind PEG-Derivate von hydriertem Ricinusöl, die z. B. unter der Bezeichnung PEG Hydrogenated Castor Oil erhältlich sind, z.B. PEG-30 Hydrogenated Castor Oil, PEG-33 Hydrogenated Castor Oil, PEG-35 Hydrogenated Castor Oil, PEG-36 Hydrogenated Castor Oil oder PEG-40 Hydrogenated Castor Oil. Erfindungsgemäß bevorzugt ist die Verwendung von PEG-40 Hydrogenated Castor Oil. Diese sind bevorzugt in einer Menge von 0,05 bis 1,5 Gew.-% enthalten, bevorzugter 0,1 bis 1,0 Gew.-%, ebenfalls bevorzugt 0,2 bis 0, 8 Gew.-% oder 0,3 bis 0,6 Gew.-%.

Die erfindungsgemäßen kosmetischen Mittel enthalten die Inhalts- bzw. Wirkstoffe in einem kosmetisch akzeptablen Träger.

Bevorzugte kosmetisch akzeptable Träger sind wässrige, alkoholische oder wässrig-alkoholische Medien mit vorzugsweise mindestens 10 Gew.-% Wasser, berechnet auf das Gesamtgewicht des Mittels.

Besonders bevorzugt enthält der erfindungsgemäße kosmetische Träger Wasser, insbesondere in der Menge, dass das kosmetische Mittel, berechnet auf das Gesamtgewicht des Mittels, mindestens 10 Gew.-%, insbesondere mindestens 20,0 Gew.-%, am bevorzugtesten mindestens 40 Gew.-% Wasser enthält.

Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Beispiele für wasserlösliche Lösungsmittel als Cosolvens sind Glycerin und/oder Ethylenglykol und/oder 1,2-Propylenglykol in einer Menge von 0 bis 30 Gew.-% bezogen auf das gesamte Mittel.

Die kosmetische Zusammensetzung der vorliegenden Erfindung kann in den für die temporäre Umformung von Haaren üblichen Formen konfektioniert sein, z. B. als Haargel, Haarspray Haarschaum oder Haarwachs. Bevorzugt ist die Konfektionierung als Haargel.

Sowohl Haarschäume als auch Haarsprays erfordern die Anwesenheit von Treibmitteln. Erfindungsgemäß sollten dafür jedoch bevorzugt keine oder nur geringe Mengen an Kohlenwasserstoffen eingesetzt werden. Dimethylether ist erfindungsgemäß ein geeignetes Treibmittel.

Die vorliegende Erfindung betrifft auch die Verwendung von erfindungsgemäßen kosmetischen Zusammensetzungen zur temporären Umformung von keratinischen Fasern, insbesondere von menschlichen Haaren, sowie ein Verfahren zur temporären Verformung von keratinischen Fasern, insbesondere menschlichen Haaren, bei dem die erfindungsgemäße kosmetische Zusammensetzung auf keratinische Fasern appliziert wird.

Tabellarische Übersicht

Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben). (Formel 1 - 5b nicht Teil der Erfindung).

|  | Formel 1 | Formel 2 | Formel 3 | Formel 4 | Formel 5 |
|---|---|---|---|---|---|
| Copolymer a) | 0,1 bis 5,0 | 0,1 bis 3,5 | 0,2 bis 1,5 | 0,3 bis 0,8 | 0,4 bis 0,6 |
| Copolymer b) | 0,1 bis 5,0 | 0,2 bis 4,0 | 0,3 bis 2,0 | 0,6 bis 1,4 | 0,8 bis 1,2 |
| Misc | add 100 | add 100 | add 100 | add 100 | add 100 |

|  | Formel 1a | Formel 2a | Formel 3a | Formel 4a | Formel 5a |
|---|---|---|---|---|---|
| Copolymer a): Acrylates / Hydroxyesters Acrylates Copolymer | 0,1 bis 5,0 | 0,1 bis 3,5 | 0,2 bis 1,5 | 0,3 bis 0,8 | 0,4 bis 0,6 |
| Copolymer b): Acrylates Copolymer (and) Water | 0,1 bis 5,0 | 0,2 bis 4,0 | 0,3 bis 2,0 | 0,6 bis 1,4 | 0,8 bis 1,2 |
| Misc | add 100 | add 100 | add 100 | add 100 | add 100 |

|  | Formel 1b | Formel 2b | Formel 3b | Formel 4b | Formel 5b |
|---|---|---|---|---|---|
| Copolymer a): Acudvne® 1000 | 0,1 bis 5,0 | 0,1 bis 3,5 | 0,2 bis 1,5 | 0,3 bis 0,8 | 0,4 bis 0,6 |
| Copolymer b): AquaStyle® SH-100 | 0,1 bis 5,0 | 0,2 bis 4,0 | 0,3 bis 2,0 | 0,6 bis 1,4 | 0,8 bis 1,2 |
| Misc | add 100 | add 100 | add 100 | add 100 | add 100 |

|  | Formel 11 | Formel 12 | Formel 13 | Formel 14 | Formel 15 |
|---|---|---|---|---|---|
| Copolymer a) | 0,1 bis 5,0 | 0,1 bis 3,5 | 0,2 bis 1,5 | 0,3 bis 0,8 | 0,4 bis 0,6 |
| Copolymer b) | 0,1 bis 5,0 | 0,2 bis 4,0 | 0,3 bis 2,0 | 0,6 bis 1,4 | 0,8 bis 1,2 |
| Carbomer | 0,02 bis 3,0 | 0,05 bis 2,0 | 0,05 bis 1,5 | 0,2 bis 1,0 | 0,4 bis 0,8 |
| Misc | add 100 | add 100 | add 100 | add 100 | add 100 |

|  | Formel 11a | Formel 12a | Formel 13a | Formel 14a | Formel 15a |
|---|---|---|---|---|---|
| Copolymer a): Acrylates / Hydroxyesters Acrylates Copolymer | 0,1 bis 5,0 | 0,1 bis 3,5 | 0,2 bis 1,5 | 0,3 bis 0,8 | 0,4 bis 0,6 |
| Copolymer b): Acrylates Copolymer (and) Water | 0,1 bis 5,0 | 0,2 bis 4,0 | 0,3 bis 2,0 | 0,6 bis 1,4 | 0,8 bis 1,2 |
| Carbomer | 0,02 bis 3,0 | 0,05 bis 2,0 | 0,05 bis 1,5 | 0,2 bis 1,0 | 0,4 bis 0,8 |
| Misc | add 100 | add 100 | add 100 | add 100 | add 100 |

|  | Formel 11b | Formel 12b | Formel 13b | Formel 14b | Formel 15b |
|---|---|---|---|---|---|
| Copolymer a): Acudyne® 1000 (Angaben als Feststoffgehalt) | 0,1 bis 5,0 | 0,1 bis 3,5 | 0,2 bis 1,5 | 0,3 bis 0,8 | 0,4 bis 0,6 |
| Copolymer b): AquaStyle® SH-100 (Angaben als Feststoffgehalt) | 0,1 bis 5,0 | 0,2 bis 4,0 | 0,3 bis 2,0 | 0,6 bis 1,4 | 0,8 bis 1,2 |
| Carbomer | 0,02 bis 3,0 | 0,05 bis 2,0 | 0,05 bis 1,5 | 0,2 bis 1,0 | 0,4 bis 0,8 |
| Misc | add 100 | add 100 | add 100 | add 100 | add 100 |

|  | Formel 21 | Formel 22 | Formel 23 | Formel 24 | Formel 25 |
|---|---|---|---|---|---|
| Copolymer a) | 0,1 bis 5,0 | 0,1 bis 3,5 | 0,2 bis 1,5 | 0,3 bis 0,8 | 0,4 bis 0,6 |
| Copolymer b) | 0,1 bis 5,0 | 0,2 bis 4,0 | 0,3 bis 2,0 | 0,6 bis 1,4 | 0,8 bis 1,2 |
| Carbomer | 0,02 bis 3,0 | 0,05 bis 2,0 | 0,05 bis 1,5 | 0,2 bis 1,0 | 0,4 bis 0,8 |

(fortgesetzt)

|  | Formel 21 | Formel 22 | Formel 23 | Formel 24 | Formel 25 |
|---|---|---|---|---|---|
| PEG-40 Hydrogenated Castor Oil | 0,05 bis 1,5 | 0,1 bis 1,0 | 0,2 bis 0,9 | 0,3 bis 0,8 | 0,4 bis 0,6 |
| Misc | add 100 | add 100 | add 100 | add 100 | add 100 |

|  | Formel 21a | Formel 22a | Formel 23a | Formel 24a | Formel 25a |
|---|---|---|---|---|---|
| Copolymer a): Acrylates / Hydroxyesters Acrylates Copolymer | 0,1 bis 5,0 | 0,1 bis 3,5 | 0,2 bis 1,5 | 0,3 bis 0,8 | 0,4 bis 0,6 |
| Copolymer b): Acrylates Copolymer (and) Water | 0,1 bis 5,0 | 0,2 bis 4,0 | 0,3 bis 2,0 | 0,6 bis 1,4 | 0,8 bis 1,2 |
| Carbomer | 0,02 bis 3,0 | 0,05 bis 2,0 | 0,05 bis 1,5 | 0,2 bis 1,0 | 0,4 bis 0,8 |
| PEG-40 Hydrogenated Castor Oil | 0,05 bis 1,5 | 0,1 bis 1,0 | 0,2 bis 0,9 | 0,3 bis 0,8 | 0,4 bis 0,6 |
| Misc | add 100 | add 100 | add 100 | add 100 | add 100 |

|  | Formel 21b | Formel 22b | Formel 23b | Formel 24b | Formel 25b |
|---|---|---|---|---|---|
| Copolymer a): Acudyne® 1000 (Angaben als Feststoffgehalt) | 0,1 bis 5,0 | 0,1 bis 3,5 | 0,2 bis 1,5 | 0,3 bis 0,8 | 0,4 bis 0,6 |
| Copolymer b): AquaStyle® SH-100 (Angaben als Feststoffgehalt) | 0,1 bis 5,0 | 0,2 bis 4,0 | 0,3 bis 2,0 | 0,6 bis 1,4 | 0,8 bis 1,2 |
| Carbomer | 0,02 bis 3,0 | 0,05 bis 2,0 | 0,05 bis 1,5 | 0,2 bis 1,0 | 0,4 bis 0,8 |
| PEG-40 Hydrogenated Castor Oil | 0,05 bis 1,5 | 0,1 bis 1,0 | 0,2 bis 0,9 | 0,3 bis 0,8 | 0,4 bis 0,6 |
| Misc | add 100 | add 100 | add 100 | add 100 | add 100 |

Unter "Misc" ist erfindungsgemäß ein kosmetischer Träger zu verstehen, insbesondere Wasser und ggf. weitere übliche Bestandteile von Stylingprodukten.

Beispiele

1. Es wurde folgendes Stylinggel hergestellt:

| Komponente/Rohstoff | Gew.-% | INCI-Bezeichnung | Hersteller |
|---|---|---|---|
| Water, demineralized | 61,476 |  |  |
| Polygel HP / Synthalen HP | 0,6 | Carbomer | 3V |
| DMDM Hydantoin 55% | 0,1 | DMDM Hydantoin |  |
| Water, demineralized | 2,754 |  |  |
| Sodium hydroxide pearls | 0,49 |  |  |
| Water, demineralized, ohne $H_2O_2$ | 29,7 |  |  |
| Acudyne® 1000 Polymer | 1 | Acrylates / Hydroxyesters Acrylates Copolymer | Dow |
| AquaStyle SH-100 | 3,3 | Acrylates Copolymer (and) Water | Ashland Inc. |
| Castor oil hydrog., 40 EO | 0,4 | PEG-40 Hydrogenated Castor Oil |  |
| Water, demineralized | 0,1 |  |  |

(fortgesetzt)

| Komponente/Rohstoff | Gew.-% | INCI-Bezeichnung | Hersteller |
|---|---|---|---|
| Parfum TEU-D-4106/6 Nutri Acai | 0,08 | | |
| **Gesamt** | <u>100</u> | | |

Die Mengenangaben in der Tabelle sind in Gew.-% des jeweiligen Rohstoffs, bezogen auf die gesamte Zusammensetzung, angegeben.

Das Haargel bildete bei Auftragen auf menschliches Haar eines transparenten Film mit sehr geringer Klebrigkeit.

Steifheit:

In eine trockene Haarsträhne (Euro-Naturhaar der Firma Kerling, 826500 Klebetresse dicht, einseitig geklebt, Gesamtlänge 150 mm, freie Länge 130 mm, Breite 20 mm, Gewicht 1,8 ± 0,2 g) wurden 850 mg einer gelförmigen Testzusammensetzung mit den Fingern einmassiert. Die mit der zu untersuchenden Testzusammensetzung behandelte Haarsträhne wird in einer Teflon-Schiene mit einem Durchmesser von 20 mm begradigt. Die präparierten Strähnen wurden anschließend über Nacht bei 21 [deg.]C und 50% relativer Luftfeuchte im Klimaraum getrocknet und konditioniert.

Die konditionierte Strähne wurde vorsichtig aus der Teflon-Schiene genommen. Die entstandene flache Strähne wurde auf die 40mm voneinander entfernten Messblöcke gelegt. Darüber wird mittig der 3PB Adapter eines Universalprüfgeräts AMETEK LF Plus der Firma AMETEK Precision Instruments Europe GmbH, Produktgruppe Lloyd montiert. Die gesamte Messung erfolgte im Klimaraum unter konstanten klimatischen Bedingungen bei 21 [deg.]C und 50% relativer Luftfeuchte.

Um standardisierte Ausgangsbedingungen zu schaffen, startete die Messung mit dem Anfahren einer Vorlast von 0,05 N. Anschließend wurde die Strähne mit einer Geschwindigkeit von 500 mm $min^{-1}$ um 15 mm gedrückt, wobei die dazu nötige Kraft gemessen wird. Nachdem die charakteristische Kraft K bei der maximalen Deformation von 15 mm aufgezeichnet wurde.

Mit dieser Messmethode lässt sich anhand der Kraft Fmax als Parameter der Steifheit des Polymerfilms und der Haltegrad des dadurch generierten Frisurenhalts messen.

Pro Testzusammensetzungen wurden 10 Strähnen erstellt und vermessen. Es wurden Steifheitswerte im Bereich von 3,0 -3,5 N erhalten, wobei es sich um arithmetische Mittel handelte.

Langzeithalt:

Die Zusammensetzung wurde mittels einer Long Lasting Hold Messung hinsichtlich ihrer formgebenden Eigenschaften überprüft. Hierzu wurden standardisierte Haarsträhnen der Fa. Kerling (Art. Nr. 826500) des Haartyps "European Natural, Farbe 6/0) von einer Länge ($L_{max}$) von 220 mm und einem Gewicht von 3,0 g eingesetzt. Zur Vorbereitung wurden die Strähnen mit einer 12,5 Gew.-%igen Natriumlaurethsulfat-Lösung gewaschen. Die Haarsträhnen wurden über Nacht in einem Trockenofen bei 318 K getrocknet.

Die Haare wurden 20 min in lauwarmen Wasser eingeweicht und dann auf ca. 50% Restfeuchte im Haare abgetupft,

750 mg der Zusammensetzung wurden auf jede der Haarsträhnen aufgebracht und einmassiert. Die Haarsträhnen wurden in eine Teflonschiene eingelegt, mittels einer Stahlrolle geglättet und über Nacht bei 21 [deg.]C und 50% getrocknet.

Die Haarsträhnen wurden nachfolgend an ihrem eine Ende in eine Haltevorrichtung eingespannt, und für eine Dauer von sechs Stunden bei 21 [deg.]C und 85% relativer Luftfeuchte gelagert. Zur Berechnung des Long Lasting Hold (LLH) wurden die aus der Haltevorrichtung herausragende Strähnenlänge vor ($L_0$) und nach ($L_t$) der Lagerung gemessen.

Der Long Lasting Hold ist ein Maß für die zeitliche Längenänderung einer mittels eines Haarverformungsmittels fixierten Haarsträhne. Je höher der LLH-Wert, desto geringer die Längenänderung der Haarsträhne unter Einfluss von Luftfeuchtigkeit in einer bestimmten Zeitdauer und desto besser der Haltegrad des Haarverformungsmittels.

Der Long Lasting Hold wurde nach folgender Formel berechnet.

$$LLH = 1 - (L_t - L_0/ L_{max})$$

Es wurde ein LLH-Werte von 59-61% ermittelt (arithmetisches Mittel der LLH-Werte von zehn Teststrähnen).

Das erfindungsgemäße Gel zeigte somit eine außergewöhnlich gute Kombination aus Langzeithalt und Steifheit.

**Patentansprüche**

1. Kosmetische Zusammensetzung zur Haarfestigung, welche enthält:

   - 0,1 bis 1,5 Gew.-% mindestens eines anionischen Acrylatcopolymers (a), welches zumindest aus folgenden Monomereinheiten aufgebaut ist: mindestens einer (Meth)Acrylsäureeinheit (a1), mindestens einer (Meth)Acrylsäurealkylestereinheit (a2) und mindestens einer (Meth)Acrylsäurehydroxyalkylestereinheit (a3), und
   - 0,3 bis 2,0 Gew.-% mindestens eines anionischen Acrylatcopolymers (b), welches zumindest aus folgenden Monomereinheiten aufgebaut ist: mindestens einer (Meth)Acrylsäureeinheit (b1), mindestens einer (Meth)Acrylsäureethylestereinheit (b2) und mindestens einer (Meth)Acrylsäureestereinheit (b3), die von der (Meth)Acrylsäureethylestereinheit (b2) verschieden ist und eine hydrophobe Gruppe als Estergruppe aufweist und
   - 0,05 bis 1,5 Gew.-% Homopolyacrylsäure.

2. Kosmetische Zusammensetzung nach Anspruch 1, wobei das anionische Acrylatcopolymer (a) ein Molekulargewicht von 130000 bis 160000 aufweist.

3. Kosmetische Zusammensetzung nach Anspruch 1 oder 2, wobei das anionische Acrylatcopolymer (a) Hydroxyethyl(meth)acrylat als Monomereinheit (a3) aufweist.

4. Kosmetische Zusammensetzung nach einem der Ansprüche 1 bis 3, wobei das anionische Acrylatcopolymer (b) einen (Meth)Acrylsäurealkylester als Monomereinheit (b3) aufweist.

5. Kosmetische Zusammensetzung einem der vorhergehenden Ansprüche, wobei das anionische Acrylatcopolymer (a) eines mit der INCI-Bezeichnung Acrylates / Hydroxyesters Acrylates Copolymer, insbesondere Acudyne™ 1000 (Dow), ist und das anionische Acrylatcopolymer (b) eines mit der INCI-Bezeichnung Acrylates Copolyme (and) Water, insbesondere AquaStyle™ SH-100 (Ashland Inc.), ist.

6. Verwendung einer kosmetischen Zusammensetzung nach einem der Ansprüche 1 bis 5 zur temporären Umformung keratinischer Fasern.

7. Verfahren zur temporären Verformung von keratinischen Fasern, insbesondere menschlichen Haaren, bei dem die kosmetische Zusammensetzung nach einem der Ansprüche 1 bis 5 auf keratinische Fasern appliziert wird.

**Claims**

1. A cosmetic composition for hair setting, containing:

   - 0.1 to 1.5 wt.% of at least one anionic acrylate copolymer (a) which is made up of at least the following monomer units: at least one (meth)acrylic acid unit (a1), at least one (meth)acrylic acid alkyl ester unit (a2), and at least one (meth)acrylic acid hydroxyalkyl ester unit (a3), and
   - 0.3 to 2.0 wt.% of at least one anionic acrylate copolymer (b) which is made up of at least the following monomer units: at least one (meth)acrylic acid unit (b1), at least one (meth)acrylic acid ethyl ester unit (b2), and at least one (meth)acrylic acid ester unit (b3) which is different from the (meth)acrylic acid ethyl ester unit (b2) and has a hydrophobic group as the ester group and
   - 0.05 to 1.5 wt.% of homopolyacrylic acid.

2. The cosmetic composition according to claim 1, wherein the anionic acrylate copolymer (a) has a molecular weight of from 130,000 to 160,000.

3. The cosmetic composition according to claim 1 or 2, wherein the anionic acrylate copolymer (a) has hydroxyethyl (meth)acrylate as monomer unit (a3).

4. The cosmetic composition according to one of claims 1 to 3, wherein the anionic acrylate copolymer (b) has a (meth)acrylic acid alkyl ester as monomer unit (b3).

5. The cosmetic composition according to one of the preceding claims, wherein the anionic acrylate copolymer (a) is a copolymer with the INCI name Acrylates / Hydroxyesters Acrylates Copolymer, in particular Acudyne™ 1000 (Dow), and the anionic acrylate copolymer (b) is a copolymer with the INCI name Acrylates Copolymer (and) Water, in particular AquaStyle™ SH-100 (Ashland Inc.).

6. The use of a cosmetic composition according to one of claims 1 to 5 for the temporary reshaping of keratin fibers.

7. A method for the temporary shaping of keratin fibers, in particular human hair, in which the cosmetic composition according to one of claims 1 to 5 is applied to keratin fibers.

**Revendications**

1. Composition cosmétique destinée à la fixation des cheveux, laquelle contient :

   - 0,1 à 1,5 % en poids d'au moins un copolymère d'acrylate anionique (a) qui comporte au moins des unités de monomères suivantes : au moins une unité d'acide (méth)acrylique (a1), au moins une unité d'ester alkylique d'acide (méth)acrylique (a2) et au moins une unité d'ester hydroxyalkylique d'acide (méth)acrylique (a3), et
   - 0,3 à 2,0 % en poids d'au moins un copolymère d'acrylate anionique (b) qui comporte au moins des motifs monomères suivants : au moins une unité d'acide (méth)acrylique (b1), au moins une unité d'ester éthylique d'acide (méth)acrylique (b2) et au moins une unité d'ester d'acide (méth)acrylique (b3), différente d'unité d'ester éthylique d'acide (méth)acrylique (b2) et comportant un groupe hydrophobe comme groupe ester, et
   - 0,05 à 1,5 % en poids d'acide homopolyacrylique.

2. Composition cosmétique selon la revendication 1, dans laquelle le copolymère d'acrylate anionique (a) a un poids moléculaire de 130000 à 160000.

3. Composition cosmétique selon la revendication 1 ou 2, dans laquelle le copolymère d'acrylate anionique (a) comporte le (méth)acrylate d'hydroxyéthyle comme unité monomère (a3).

4. Composition cosmétique selon l'une des revendications 1 à 3, dans laquelle le copolymère d'acrylate anionique (b) comporte un ester alkylique d'acide (méth)acrylique comme unité monomère (b3).

5. Composition cosmétique selon l'une des revendications précédentes, dans laquelle le copolymère d'acrylate anionique (a) est un copolymère portant la npmenclature INCI Acrylates/Hydroxyester Acrylates Copolymer, notamment Acudyne™ 1000 (Dow) et le copolymère a anionique (b) est un copolymère portant la nomenclature INCI Acrylates Copolymer (and) Water, notamment AquaStyle™ SH-100 (Ashland Inc.).

6. Utilisation d'une composition cosmétique selon l'une des revendications 1 à 5 pour la mise en forme temporaire des fibres kératinique.

7. Procédé de mise en forme temporaire de fibres de kératine, en particulier de cheveux humains, dans lequel la composition cosmétique selon une des revendications 1 à 5 est appliquée sur des fibres kératinique.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1719499 B1
- EP 1719500 B1
- EP 1726331 A
- WO 2012054278 A2